# EUROPÄISCHE PATENTSCHRIFT

(11) **EP 0 908 449 B1**
(45) Veröffentlichungstag und Bekanntmachung des Hinweises auf die Patenterteilung: **21.11.2001**
(21) Anmeldenummer: 98118349.4
(22) Anmeldetag: 28.09.1998
(51) Int. Cl.: C07C 403/24, C07C 403/14, C07C 403/20, C07C 67/343, C07C 69/602, C07C 1/34, C07C 11/21, C07C 45/51, C07C 47/21

(54) **Herstellung von Carotinoiden**
Preparation of carotenoids
Préparation de caroténoides

(30) Priorität: 03.10.1997 EP 97117192
(43) Veröffentlichungstag der Anmeldung: 14.04.1999
(73) Patentinhaber: F. HOFFMANN-LA ROCHE AG, 4070 Basel (CH)
(72) Erfinder: Kreienbühl, Paul, 4125 Riehen (CH); Rudin, Peter, 4058 Basel (CH); Rudolph, Werner, 4125 Riehen (CH)

(56) Entgegenhaltungen:
- DE-A- 19 509 955
- US-A- 3 496 170
- US-A- 3 517 067

## Beschreibung

Carotinoide sind eine in der Natur weitverbreitete Stoffklasse mit sehr interessanten Eigenschaften. Deshalb besteht ein grosses Interesse an technisch realisierbaren Synthesen. Beim Aufbau der jeweiligen Polyenkette spielt die Wittigreaktion eine sehr grosse Rolle. Sie ist sehr eingehend untersucht und auch technisch angewandt worden.

Carotinoide (Zwischen- und Endprodukte), z. B. Carotine und Xanthophylle, sind in der normalerweise gewünschten (all E)-Konfiguration in den meisten Lösungsmitteln wenig löslich. Aromatische Kohlenwasserstoffe und besonders halogenierte, niedere aliphatische Kohlenwasserstoffe, wie Chloroform und Methylenchlorid, nehmen als Lösungsmittel wegen ihres guten Lösevermögens für Carotinoide eine gewisse Sonderstellung ein und wurden bisher zu diesem Zweck bevorzugt eingesetzt. Nachteilig ist jedoch heutzutage die schwindende Akzeptanz solcher Lösungsmittel aus bekannten und berechtigten Gründen. Dazu kommt eine für Carotinoide typische Eigenschaft vor, Lösungsmittel in nichtstöchiometrischen Mengen einzuschliessen. Rückstände von den obengenannten Lösungsmitteln unterliegen strengen Vorschriften, die zudem nicht überall gleich sind. Die thermische Stabilität von Carotinoiden ist jedoch häufig kritisch, so dass die Entfernung der eingeschlossenen Lösungsmittelspuren schwierig wird und nur unter Inkaufnahme von beginnender Zersetzung überhaupt realisiert werden kann.

Die bekannten technischen Verfahren lassen sich in folgende Untergruppen einteilen:
1) Homogene Verfahren, bei denen die Edukte und das Produkt während der ganzen Reaktion in Lösung bleiben; die Isolierung des Endproduktes geschieht durch Ausfällung, typischerweise verbunden mit einem Isomerisierungsschritt.
2) Zweiphasige Verfahren, bei denen die Edukte in einem organischen Lösungsmittel gelöst werden, die notwendige Base jedoch in wässriger Lösung zugegeben wird. Zur Aufarbeitung werden die Phasen voneinander getrennt und die organische Phase ähnlich wie in 1) aufgearbeitet.
3) Heterogene Verfahren, bei denen die Edukte ungelöst eingesetzt werden. Während der Reaktion entsteht jedoch mindestens zeitweise eine klare Lösung. Andererseits gibt es auch umgekehrte Ausführungsformen, bei welchen die Reaktion als Lösung in Gang gebracht wird und das Produkt während oder nach der eigentlichen Reaktion ausfällt.

Die Wittigreaktion wird generell basisch oder wenigstens in Anwesenheit einer säurebindenden Substanz durchgeführt. Als Abfall der Wittigreaktion entsteht immer ein Äquivalent Triphenylphosphinoxid, welches aus dem Reaktionsgemisch abgetrennt werden muss. Die Wahl des Lösungsmittels beeinflusst massgeblich die Aufarbeitung.

Die wichtigste generell auftretende Nebenreaktion bei einer Wittigreaktion ist die Hydrolyse des Phosphoniumsalzes : siehe das nachfolgende Reaktionsschema.

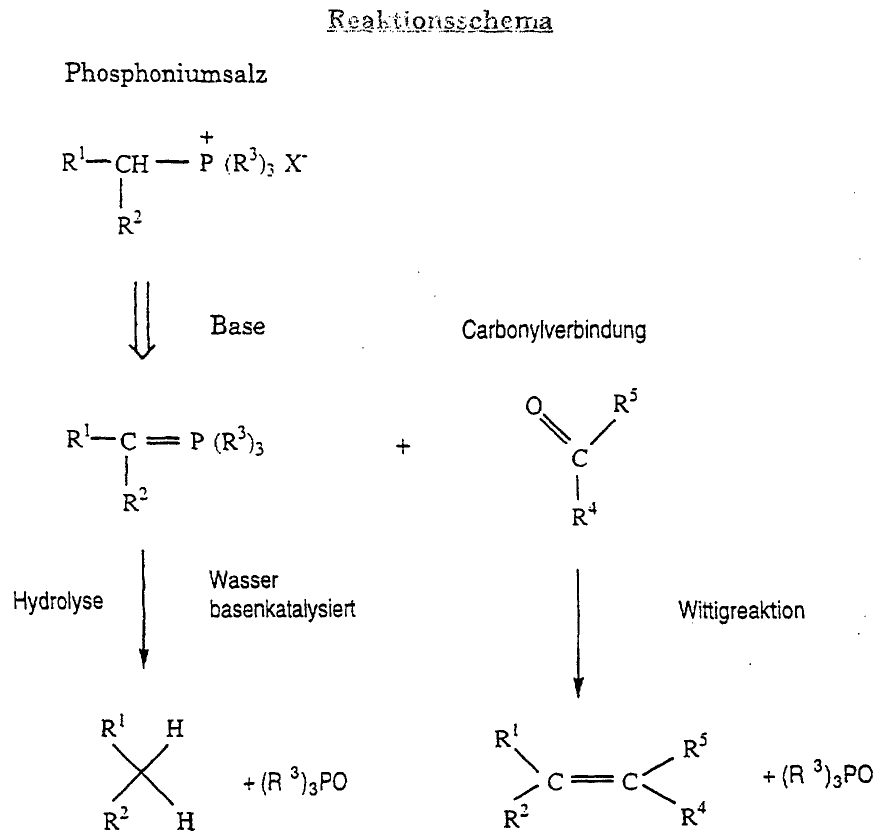

In diesem Schema bedeuten R¹ und R² bzw. R⁴ und R⁵ jeweils Wasserstoff oder eine gegebenenfalls substituierte Alkyl-, Alkenyl- oder Aryl-, z.B. Phenyl-, gruppe, wobei höchstens eins der beiden Symbole R¹ und R² bzw. R⁴ und R⁵ für Wasserstoff stehen kann; R³ bedeutet Aryl, vorzugsweise Phenyl, und X⁻ ein Anion, beispielsweise Chlorid, Bromid, Sulfat oder Acetat. Infolge der Bevorzugung von R³ bedeutet (R³)₃PO vorzugsweise Triphenylphosphinoxid. Bei den Carbonylverbindungen handelt es sich insbesondere um dem auf dem Gebiet der Carotinoidchemie tätigen Fachmann bestens bekannte Aldehyde, einschliesslich Dialdehyde, und Ketone, von denen die Aldehyde vorzugsweise eingesetzt werden. Ein Beispiel eines typischerweise eingesetzten Dialdehyds ist das 2,7-Dimethyl-2,4,6-octatrien-1,8-dial ("C₁₀-Dial"). Unter dem Phosphoniumsalz ist ebenfalls insbesondere eins zu verstehen, das typischerweise zur Synthese von Carotinoiden verwendet wird und daher dem Fachmann bestens bekannt ist. Viele Beispiele der einschlägigen Wittigreaktion sind in den Lehrbüchern "Carotenoids", Ed. Otto Isler, Birkhäuser Verlag Basel 1971, und "Carotenoids, Volume 2: Synthesis", Ed. G. Britton, S. Liaaen-Jensen und H. Pfander, Birkhäuser Verlag Basel Boston Berlin, 1996, enthalten.

Zur Vermeidung der oben erwähnten und im Schema dargestellten Nebenreaktion können je nach Substrat die Basenstärke, der Wassergehalt und/oder die Reaktionstemperatur in sehr weitem Rahmen variiert werden. So ist es bekannt, Wittigreaktionen in Wasser als Lösungsmittel durchzuführen, wogegen in anderen Fällen die Reaktion streng wasserfrei geführt werden muss. Die Hydrolyse wird mit steigender Basenstärke und/oder Temperatur relativ schneller, so dass häufig Temperaturen von -30°C bis Raumtemperatur bevorzugt sind; andererseits gibt es Fälle, bei denen mit Vorteil erhöhte Temperatur verwendet wird. Wiederum abhängig vom Substrat sind bei höheren Temperaturen für Carotinoide Isomerisierungsvorgänge typisch.

Die Wittigreaktion kann in praktisch allen nichtsauren Lösungsmitteln durchgeführt werden. Selbst Aceton, welches selber mit Phosphoniumsalzen reagieren kann, wurde vereinzelt erfolgreich eingesetzt.

Die oben erwähnten bekannten Ausführungsformen der Wittigreaktion haben Nachteile. Die vom toxikologischen Standpunkt aus bevorzugten Lösungsmittel sind Wasser, niedere Alkanole, Aceton und Ester von Essigsäure und anderen organischen Säuren. Diese Lösungsmittel, von denen die organischen gegebenenfalls mit Wasser kombiniert werden können, weisen in der Regel nur ein sehr ungünstiges Lösevermögen für Carotinoide auf. Dadurch wird häufig die heterogene Reaktionsführung angewandt. Ein aktuelles Beispiel dafür befindet sich in der europäischen Patentpublikation (EP) 0 733 619. Allerdings werden gerade in dieser EP die Grenzen sichtbar. Um mit Methanol oder Ethanol noch eine praktikable Konzentration an Edukten zu erreichen, müssen beide Komponenten vorgängig warm gelöst werden und (als Kunstgriff) ein Edukt als Isomerengemisch verwendet werden. Die eigentliche Wittigreaktion erfordert in diesem Beispiel tiefere Temperaturen, weshalb zuerst wieder abgekühlt werden muss.

Die US-Patentschrift 3 517 067 offenbart die Herstellung gewisser quaternärer Phosphoniumsalze sowie im Falle des Retinyltriphenylphosphoniumsalzes dessen Verwendung als Zwischenprodukt bei der Synthese von β-Carotin durch die Wittigreaktion. Im diesbezüglichen Absatz der allgemeinen Beschreibung (Spalte 1, Zeilen 43 - 62) sind keine Details über die Reaktionsführung erwähnt. Erst in Beispiel 2 wird die Synthese von β-Carotin auf diese Weise exemplifiziert, und in den Beispielen 3 - 6 sind weitere derartige Synthesen exemplifiziert, zum Teil unter Bezugnahme auf Beispiel 2 für die Reaktionsführung: zumindest die Carbonylverbindung, d.h. Retinal bzw. ein Hydrochinonkomplex davon, befindet sich in einer Aufschlämmung, wobei allerdings der Grad der Auflösung nicht angegeben ist. Darüber hinaus handelt es sich bei deiser Wittigreaktion nicht um eine sogenannte "doppelte Wittigreaktion"; die Reaktion ist vielmehr eine einfache (einstufige) Wittigreaktion.

Die US-Patentschrift 3 496 170 offenbart in der allgemeinen Beschreibung vor allem eine Definition von neuen Carotinoiden, eine Auflistung von Beispielen dieser Carotenoide sowie eine Angabe zu deren Verwendung. Die Herstellung der Carotinoide ist knapp abgehandelt mit dem Hinweis, dass die Synthese durch Kopplung von geeigneten, kommerziell erhältlichen oder nach an sich bekannten Methoden hergestellten Zwischenprodukten gemäss der Wittigreaktion erfolgt (Spalte 3, Zeilen 34 - 38). Die Beispiele veranschaulichen allerdings spezifische Verfahren nach Wittig, wobei insbesondere Beispiele 2, 16 - 18, 24 und 25 als Ausgangsmaterial jeweils eine Aufschlämmung der beiden Ausgangsmaterialien Phosphoniumsalz und Carbonylverbindung aufweisen. Auch in diesem Fall gibt es unter diesen Beispielen keinen Hinweis auf den Auflösungsgrad. Ferner betreffen sämtliche Beispiele einfache, einstufige - und nicht doppelte - Wittigreaktionen.

Gegenstand der vorliegenden Erfindung ist ein chemisch und technisch breit anwendbares Verfahren, welches die Herstellung von Carotinoiden (sowohl Zwischen- als auch Endprodukten) unterschiedlichster Struktur unter Vermeidung der oben erwähnten Probleme ermöglicht. Dieses Verfahren ist ein Verfahren zur Herstellung von Carotinoiden durch die Wittigreaktion eines symmetrischen Dialdehyds mit zwei Aequivalenten eines Phosphoniumsalzes und ist dadurch gekennzeichnet, dass man eine zur Herstellung eines Carotinoids angewandte Wittigreaktion in einem polaren Reaktionsmedium derart durchführt, dass weder alle Reaktionsteilnehmer noch das so hergestellte Carotinoid im Reaktionsmedium signifikant gelöst sind, indem je nach Reaktionsteilnehmer bzw. Produkt (Carotinoid) sich höchstens 10% seines Gewichtes in Lösung befinden.

Als jeweiliges polares Reaktionsmedium eignet sich insbesondere ein polares Lösungsmittel oder Lösungsmittelgemisch, welches bei Zugabe von Wasser in einer Menge bis zu etwa 30 Vol.-% einphasig bleibt und das anfallende Triarylphosphinoxid gut zu lösen vermag. Besonders geeignet zu diesem Zweck sind niedere Alkanole und Aceton, welche als alleinige Lösungsmittel oder als Gemische untereinander und/oder mit Wasser eingesetzt werden können. Im Falle der Verwendung dieser besonders geeigneten polaren Lösungsmittel können darüber hinaus auch zusätzliche polare Lösungsmittel eingesetzt werden. Als solche kommen beispielsweise polare Ester, insbesondere die Methyl und Ethylester der Ameisensäure, Essigsäure oder Kohlensäure, Methylethylketon, tert.-Butylmethylether und Dimethylformamid in Frage. Auch diese zusätzlichen polaren Lösungsmittel können als Gemische untereinander eingesetzt werden. Auf jeden Fall soll ein aus mehreren der erwähnten polaren Lösungsmittel bestehendes Reaktionsmedium einphasig (homogen) sein.

Das erfindungsgemässe Verfahren wird derart durchgeführt, dass weder die Reaktionsteilnehmer, d.h. die jeweilige Carbonylverbindung (der symmetrisch Dialdehyd) und das jeweilige Phosphoniumsalz, noch das so hergestellte Carotinoid im Reaktionsmedium signifikant gelöst sind, hingegen das in der Reaktion anfallende Triarylphosphinoxid, insbesondere Triphenylphosphinoxid, in Lösung bleibt. Infolge des so durchgeführten Verfahrens kann man überraschenderweise gute Ausbeuten und Endproduktqualität erzielen.

Im Rahmen des oben definierten erfindungsgemässen Verfahrens ist der Begriff "weder/noch--signifikant gelöst" so zu verstehen, dass im Verfahren zu keinem Zeitpunkt der Reaktion und der Aufarbeitung, einschliesslich der Reinigung, je eine klare Lösung vorliegt. Mindestens ein Edukt, ein allfälliges Zwischenprodukt und das erwünschte Endprodukt liegen als Suspension oder Aufschlämmung (slurry) vor. Je nach Reaktionsteilnehmer bzw. Produkt (Carotinoid) befinden sich höchstens 10% seines Gewichtes in Lösung. Typischerweise beträgt dieser Grad der Auflösung nur zwischen 0,5 und 2 Gewichtsprozent. In gewissen Fällen kann dieser sogar noch viel niedriger als 0,5 Gewichtsprozent sein.

Ferner ist unter dem Ausdruck "1 bis 6 Kohlenstoffatome aufweisendes Alkanol" ein Alkanol zu verstehen, das 1 bis 6 Kohlenstoffatome, insbesondere 1 bis 4 Kohlenstoffatoms, aufweist; Beispiele davon sind Methanol, Ethanol, n-Propanol und Isopropanol. Ein Gemisch mehrerer solcher Alkanole untereinander bzw. ein Gemisch eines oder mehrerer solcher Alkanole mit Aceton kann im Rahmen der vorliegenden Erfindung eine beliebige Anzahl dieser polaren Lösungsmittel und ein beliebiges Mischungsverhältnis aufweisen. Auch im Falle der Anwesenheit von Wasser (bei den wässrigen Gemischen eines oder mehrerer niederer Alkanole und/oder von Aceton) kann das Mischungsverhältnis unter den genannten polaren organischen Lösungsmitteln beliebig variieren. Im letzteren Fall beträgt der Volumenanteil des Wassers gegenüber dem Gesamtvolumen des wässrigorganischen Lösungsmittels normalerweise nicht mehr als etwa 30%. vorzugsweise bis zu 20%. Bei dem Methyl- oder Ethylester von Kohlensäure handelt es sich um Dimethylcarbonat bzw. Diethylcarbonat. Unter allen in Frage kommenden polaren Lösungsmitteln sind Methanol, Ethanol, n-Propanol und Isopropanol bevorzugt.

Das erfindungsgemässe Verfahren funktioniert, wenn mit einem symmetrischen Dialdehyd in einem Schritt zwei Aequivalente eines Phosphoniumsalzes umgesetzt werden, ohne dass man das Zwischenprodukt isoliert. Diese Arbeitsweise wird im weiteren als "doppelte Wittigreaktion" bezeichnet.

Ansonsten gelten die normalen Reaktionsbedingungen, bei denen doppelte Wittigreaktionen durchgeführt werden, und zwar in bezug auf Reaktionstemperaturen, -drücke, -dauer usw. Auch die Aufarbeitung des nach Beendung der Reaktion erhaltenen Gemisches, um das Produkt zu isolieren, und die gewünschtenfalls durchgeführte Reinigung des Produktes, bedürfen keiner besonderen Massnahmen.

Das erfindungsgemässe Verfahren eignet sich in Prinzip für alle doppelten Wittigreaktionen zur Herstellung von Carotinoiden, u.a. zur Herstellung von:
Zeaxanthin ausgehend von [5-(4-Hydroxy-2,6,6-trimethyl-1-cyclohexenyl)-3-methyl-2,4-pentadienyl]triphenylphosphoniumchlorid und 2,7-Dimethyl-2,4,6-octatrien-1,8-dial (C₁₀-Dial);
Lycopin ausgehend von [3,7,11-Trimethyl-dodecyl-2,4,6,10-tetraenyl]triphenylphosphoniumchlorid oder -acetat, insbesondere dem (2 E/Z, 4 E, 6 E,Z)-Isomeren, und C₁₀-Dial;
Carotin ausgehend von [3-Methyl-5-(2,6,6-trimethyl-1-cyclohexenyl)-2,4-pentadienyl]triphenylphosphoniumchlorid, insbesondere dem (2E, 4E)-Isomeren, und C₁₀-Dial;
Canthaxanthin ausgehend von [5-(2,6,6-Trimethyl-3-oxo-1-cyclohexenyl)-3-methyl-2,4-pentadienyl]triphenylphosphoniumchlorid und C₁₀-Dial;
Astaxanthin ausgehend von [5-(4-Hydroxy-2,6,6-trimethyl-3-oxo-1-cyclohexenyl)-3-methyl-2,4-pentadienyl]triphenylphosphoniumbromid, insbesondere dem (2E,4E)-Isomeren, und C₁₀-Dial;
Crocetindiethylester ausgehend von [3-Ethoxycarbonyl-but-2-enyl]triphenylphosphoniumchlorid und/oder -bromid, insbesondere dem (E)-Isomeren, und C₁₀-Dial;
sowie Diapo-4,4'-carotindial ausgehend vom Dimethylacetal von [7-Formyl-3,7dimethyl-octa-2,4,6-trienyl]triphenylphosphoniumchlorid und C₁₀-Dial.

Das erfindungsgemässe Verfahren zeichnet sich durch grosse, praktische Vorteile aus:
- Es ist nun möglich, viel konzentrierter zu arbeiten (weniger Lösungsmittel als bisher verwenden zu müssen); die Konzentration wird erst durch die Abnahme der Rührbarkeit begrenzt. Dadurch werden Raum/Zeit-Ausbeuten wesentlich verbessert. Zudem wird die Regenerierung des Lösungsmittels vereinfacht, besonders auch deshalb, weil auf den Zusatz von löslichkeitserhöhenden Lösungsmitteln, wie beispielsweise Tetrahydrofuran und Methylenchlorid, verzichtet werden kann.
- Optimal einfache Reaktionsführung, ungeachtet davon, ob die Reaktion als batch- oder als kontinuierliches Verfahren erfolgen soll.
- Vollständiger Verzicht auf halogenierte Lösungsmittel und Beschränkung auf die toxikologisch unbedenklichen polaren Lösungsmittel, z.B. die bevorzugten Methanol, Ethanol, n-Propanol und Isopropanol.

Die Reaktionsführung gemäss der vorliegenden Erfindung widerspricht der klassischen Lehrmeinung, wonach eine gute Ausbeute und hohe Produktequalität nur zu erwarten ist, wenn die Edukte vollständig in Lösung gegangen sind, bevor das Produkt auszufallen beginnt. Für Carotinoide ist es um so überraschender, da diese Stoffklasse zu nichtstöchiometrischen Einschlüssen neigt und sich carotinoide Nebenprodukte wegen der gemeinsamen Strukturmerkmale selbst durch Umkristallisation nicht befriedigend abtrennen lassen.

Für viele kommerziell wichtige Carotinoide wird das Aufbauschema zwei C₁₅-Wittigsalze + C₁₀-Dial in einer doppelten Wittigreaktion verwendet. Nachteilig an diesem Syntheseweg war vor allem die ungenügende Löslichkeit dieser Komponenten in fast allen nichthalogenierten Lösungsmitteln. In niederen Alkanolen ist auch die Löslichkeit des C₂₅-Zwischenproduktes gering, und die Produkte sind ohnehin kaum löslich. Es ist deshalb sehr überraschend, dass die konsequent heterogene Reaktionsführung sich weder auf die Ausbeute noch auf die Qualität des Produktes nachteilig auswirkt.

Angesichts der dargestellten Vielfalt der Wittigreaktion ist es ausserdem sehr überraschend, dass es bei der Synthese von Carotinoiden unterschiedlichster Struktur (von C₂₀ bis C₅₀) eine einheitliche Reaktionsführung so erfolgreich ist.

Die vorliegende Erfindung wird anhand der nachfolgenden Beispiele veranschaulicht:

### Beispiel 1

### Herstellung von (all-E)-Zeaxanthin

In einem mit Innenthermometer, Rührer und Rückflusskühler ausgestatteten 500 ml-Vierhalssulfierkolben wurden 32,88 g (95,8%ig; 2,03 Aeq.) [5-(4-Hydroxy-2,6,6-trimethyl-1-cyclohexenyl)-3-methyl-2,4-pentadienyl]-triphenylphosphoniumchlorid und 4,92 g (1 Aeq.) 2,7-Dimethyl-2,4,6-octatrien-1,8-dial (C₁₀-Dial) in 490 ml Ethanol vorgelegt und unter Rühren auf -10°C gekühlt. In die resultierende gelbliche Suspension wurde unter Rühren bei-10°C eine Lösung von 3,60 g Natriumhydroxid in 50 ml Ethanol zugetropft und anschliessend das Reaktionsgemisch eine Stunde bei -10°C gerührt. Nach Erwärmen auf etwa 80°C Innentemperatur innerhalb von 30 Minuten wurde während der folgenden 17 Stunden die Produktsuspension bei der Rückflusstemperatur erhitzt. Anschliessend wurde auf 0°C abgekühlt, eine Stunde weitergerührt und das so erhaltene Zeaxanthin durch Abnutschen isoliert. Nun wurde der Filterkuchen mit Ethanol gut gewaschen und anschliessend mit Wasser vom Neutralsalz befreit.

Das Kristallisat wurde unter vermindertem Druck etwa 16 Stunden bei 100°C/40 mbar (4 kPa) getrocknet.

Auf diese Weise erhielt man 16,0 g kristallines (all-E)-Zeaxanthin mit einem Gehalt an Zeaxanthin gemäss Hochdruckflüssigchromatographie (HPLC) von mehr als 99%. Die Ausbeute betrug 93,7% (bezogen auf C₁₀-Dial).

### Beispiele 2-12

Analog der in Beispiel 1 beschriebenen Arbeitsweise wurden verschiedene weitere Wittigreaktionen durchgeführt, deren Details in der nachfolgenden Tabelle zusammengefasst sind. In jedem Beispiel wurde das C₁₀-Dial verwendet, und zwar in einer Ansatzgrösse von 30 mmol. Die Tabelle beinhaltet verschiedene Abkürzungen, deren Bedeutungen in der nach der Tabelle erscheinenden Legende angegeben sind (konventionelle chemische und sonstige geläufige Bezeichnungen bedürfen keiner näheren Erläuterung).

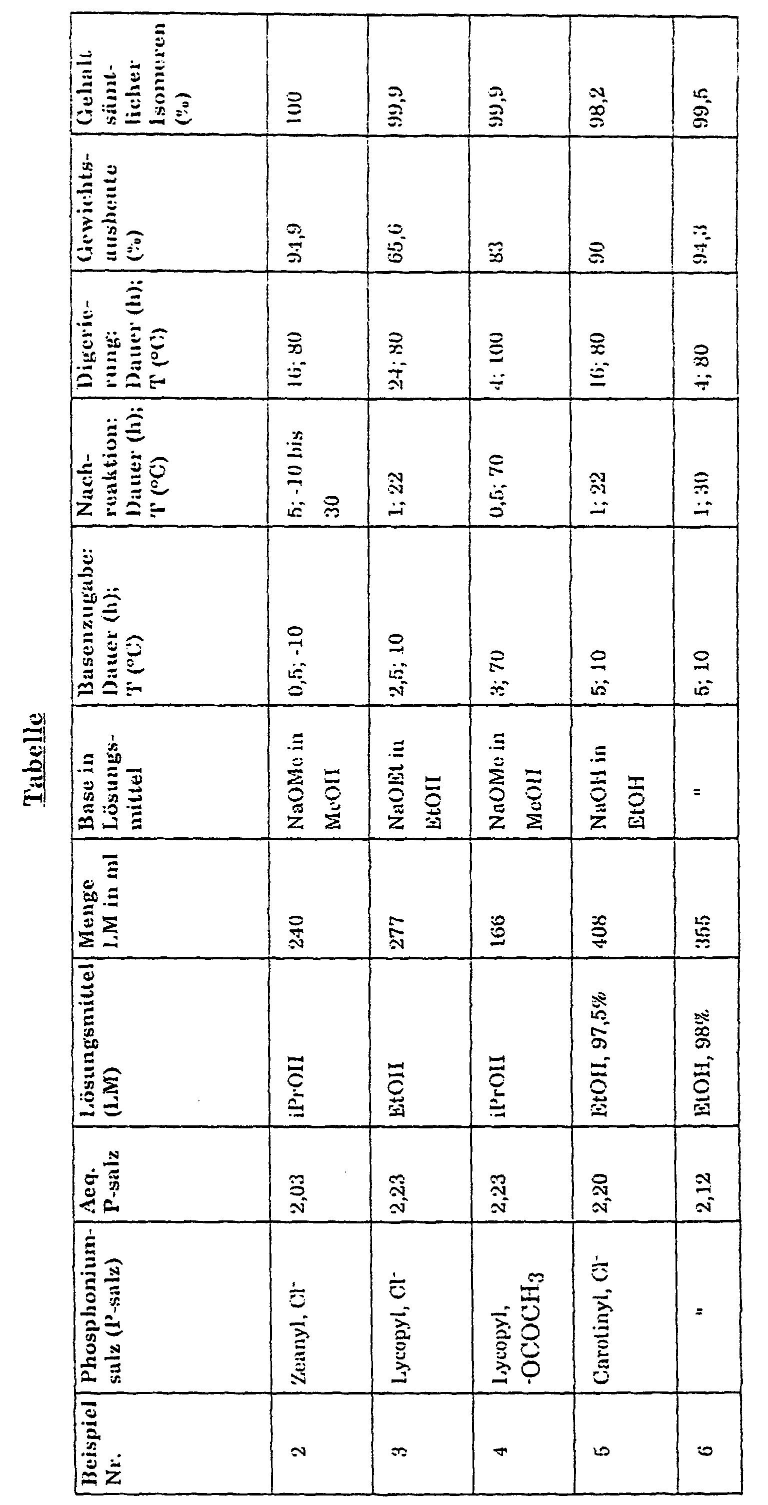

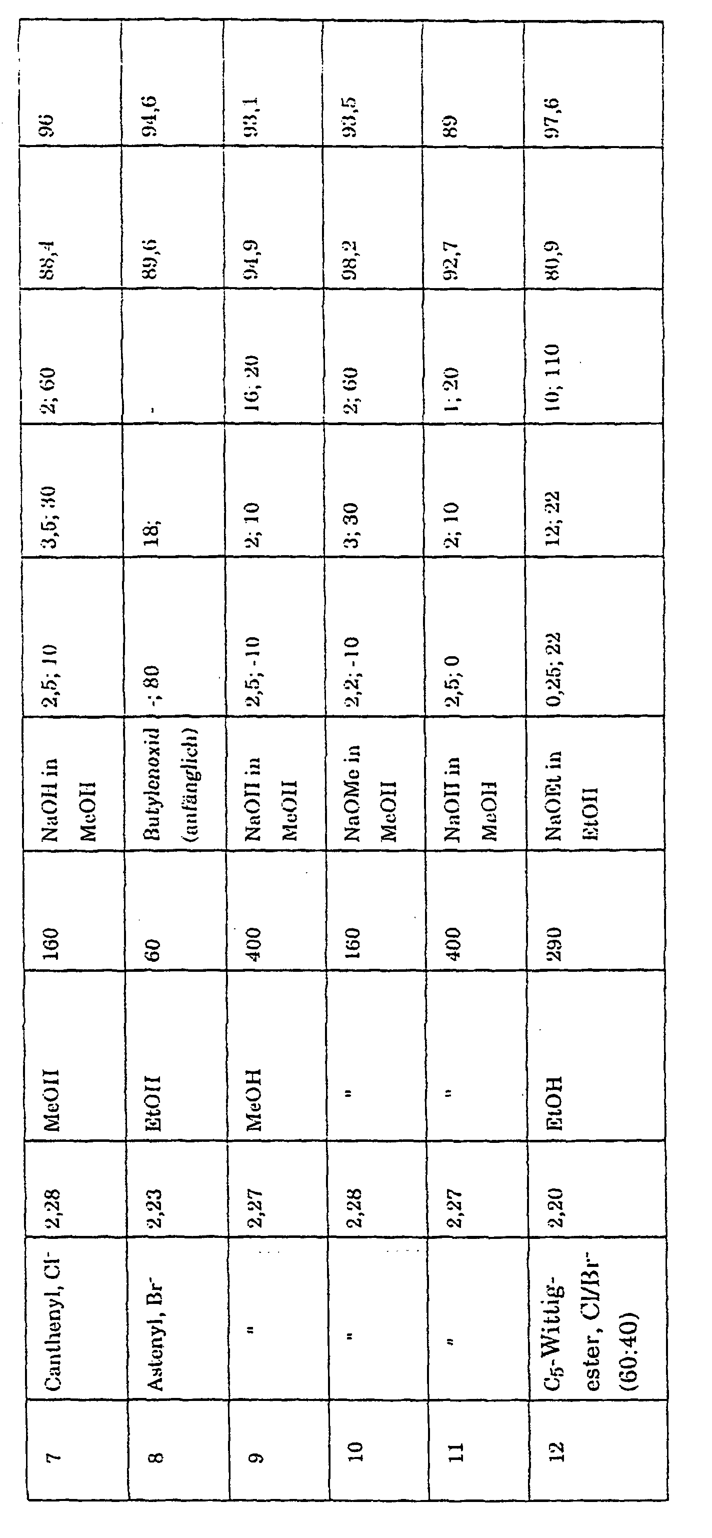

### Legende

- Zeanyl:: [5-(4-Hydroxy-2,6,6-trimethyl-1-cyclohexenyl)-3-methyl-2,4-pentadienyl]triphenylphosphonium
- Lycopyl:: [(2E/Z,4E,6E,Z)-3,7,11-Trimethyl-dodeca-2,4,6,10-tetraenyl]triphenylphosphonium
- Carotinyl:: [(2E,4E)-3-Methyl-5-(2,6,6-trimethyl-1-cyclohexenyl)-2,4-pentadienyl]triphenylphosphonium
- Canthenyl:: [5-(2,6,6-Trimethyl-3-oxo-1-cyclohexenyl)-3-methyl-2,4-pentadienyl]triphenylphosphonium
- Astenyl:: [(2E,4E)-5-(4-Hydroxy-2,6,6-trimethyl-3-oxo-1-cyclohexenyl)-3-methyl-2,4-pentadienyl]triphenylphosphonium
- C₅-Wittigester:: [(E)-3-Ethoxycarbonyl-but-2-enyl]triphenylphosphonium
- MeOH, EtOH, iPrOH:: Methanol, Ethanol (absolut), Isopropanol
- NaOMe, NaOEt:: Natriummethoxid, Natriumethoxid

### Beispiel 13

### Herstellung von Crocetindiethylester

In einem mit mechanischem Rührer, Thermometer, Tropftrichter und Stickstoffbegasung ausgestatteten 2,5 1-Vierhalskolben wurden 16,45 g (100 mmol) 2,7-Dimethyl-2,4,6-octatrien-1,8-dial und 93,5 g (220 mmol) [3-Ethoxycarbonyl-but-2-enyl]triphenylphosphoniumchlorid in 750 ml absolutem Ethanol suspendiert. Bei Raumtemperatur tropfte man unter intensivem Rühren innerhalb von 12 Minuten 220 ml (220 mmol) einer Natriumethylatlösung (1,0M in Ethanol) zu. Das Reaktionsgemisch wurde etwa 12 Stunden bei Raumtemperatur unter Stickstoffbegasung nachgerührt.

Die resultierende rote Suspension wurde mit etwa 2 ml konzentrierter Essigsäure versetzt. Anschliessend destillierte man 370 ml Ethanol aus dem Reaktionsgemisch mit einem Oelbad bei 100-110°C ab und gab darauffolgend 400 ml ionenarmes Wasser zu. Die Suspension wurde dann weitere 10 Stunden am Rückfluss (Innentemperatur 82°C), anschliessend 2 Stunden in einem Eisbad gerührt (Innentemperatur 3-4°C). Der Niederschlag wurde auf einer Glasnutsche abgetrennt und mit wässrigem, eisgekühltem Ethanol (60% in Wasser) gewaschen. Nach etwa 16-stündigem Trocknen im Wasserstrahlvakuum bei 50°C erhielt man 34 g Crocetindiethylester in roter, semikristalliner, etwas klebriger Form (Rohprodukt; Ausbeute etwa 88,5% der Theorie).

Obiges Rohprodukt wurde in 175 ml wässrigem Ethanol (60% in Wasser) suspendiert und bei 21 bar (2,1 kPa) Druck unter Stickstoffbegasung 10 Stunden bei 110°C isomerisiert. Das kristalline Produkt wurde im Wasserstrahlvakuum abgenutscht und wie zuvor mit wässrigem Ethanol gewaschen. Nach etwa 16-stündigem Trocknen im Wasserstrahlvakuum bei 50°C erhielt man 31,1 g Crocetindiethylester als rotes Kristallisat, Smp. 208-209°C.
Ausbeute 80,9% der Theorie;
HPLC: 90,4% (all-E)-Isomer;
UV (E₁¹): 3052 cm⁻¹ (max. bei 433 nm).

### Beispiel 14

### Herstellung von Diapo-4,4'-carotindial

Zur Herstellung des Dimethylacetals von [7-Formyl-3,7-dimethyl-octa-2,4,6-trienyl]triphenylphosphoniumchlorid wurden in einem mit Rührer, Thermometer, Kühler, automatischer Dosierungsvorrichtung (Dosimat) und Argonbegasung ausgestatteten 2,5 1-Vierhalssulfierkolben 45,06 g (0,099 Mol) dieses Phosphoniumsalzes unter Argonbegasung vorgelegt. Man schlämmte dann den Kolbeninhalt in 190 ml Methanol auf und versetzte die Suspension unter Rühren bei Raumtemperatur mit 12,94 ml (0,1188 Mol) Orthoameisensäure-methylester. Nach Zugabe von 1,86 ml einer 4,59%igen Lösung von p-Toluolsulfonsäure in Methanol, wurde bei Raumtemperatur gerührt. Die Reaktionslösung blieb dabei braun-orange. Nach der Reaktion wurde die Lösung mit 0,210 ml einer 29,37%igen Lösung von Natriummethylat (enthaltend 0,0014 Mol Base) neutralisiert.

Zur Durchführung der Wittigreaktion wurden nach der Acetalisierung zur obigen Reaktionslösung bei Raumtemperatur 7,39 g (0,045 Mol) 2,7-Dimethyl-2,4,6-octatrien-1,8-dial und 600 ml Isopropanol zugegeben. Es entstand eine orange Suspension. Zu dieser Suspension wurden nun bei Raumtemperatur innerhalb von 30 Minuten 20,03 ml der 29,37%igen Lösung von Natriummethylat (enthaltend 0,0495 Mol Base) zudosiert. Dabei stieg die Temperatur auf etwa 27°C, und die Suspension färbte sich dunkelbraun. Nun liess man das Reaktionsgemisch noch 1,5 Stunden bei Raumtemperatur nachreagieren. Das nun entstandene Acetal wurde innerhalb von 30 Minuten mit 395 ml 0,5N Schwefelsäure entacetalisiert. Danach wurden zur Neutralisation 395 ml 0,5N wässrige Natriumhydroxidlösung unter Rühren zugegeben. Nach etwa 90 minütigem Rühren wurde der braune Kristallbrei genutscht und der Filterkuchen der Reihe nach mit Isopropanol und entionisiertem Wasser gewaschen. Die resultierenden schwarz-braunen Kristalle wurden etwa 16 Stunden bei 50°C im Vakuumgetrockenschrank bei 20-30 mbar (2-3 kPa) getrocknet. Auf diese Weise erhielt man in einer Gewichtsausbeute von mehr als 99% 19,26 g Diapo-4,4'-carotindial als schwarz-braune Kristalle. Die Zusammensetzung des Rohproduktes gemäss HPLC betrug etwa 72% des (all-E)- und etwa 19% eines (Z)-Isomeren.

## Patentansprüche

1. Verfahren zur Herstellung von Carotinoiden durch die Wittigreaktion eines symmetrischen Dialdehyds in einem Shritt mit zwei Aequivalenten eines Phosphoniumsalzes **dadurch gekennzeichnet, dass** man die Wittigreaktion in einem polaren Reaktionsmedium derart durchführt, dass weder alle Reaktionsteilnehmer noch das so hergestellte Carotinoid im Reaktionsmedium signifikant gelöst sind, indem je nach Reaktionsteilnehmer bzw. Produkt (Carotinoid) sich höchstens 10% seines Gewichtes in Lösung befinden.

2. Verfahren nach Anspruch 1, **dadurch gekennzeichnet, dass** als Reaktionsmedium ein polares Lösungsmittel oder Lösungsmittelgemisch, welches bei Zugabe von Wasser in einer Menge bis zu etwa 30 Vol.-% einphasig bleibt und das anfallende Triarylphosphinoxid gut zu lösen vermag, verwendet wird.

3. Verfahren nach Anspruch 1, **dadurch gekennzeichnet, dass** man als polare Lösungsmittel 1 bis 6 Kohlenstoffatome aufweisende Alkanole und Aceton verwendet, welche als alleinige Lösungsmittel oder als Gemische untereinander und/oder mit Wasser eingesetzt werden.

4. Verfahren nach Anspruch 3, **dadurch gekennzeichnet, dass** jeweils ein zusätzliches polares Lösungsmittel eingesetzt wird, das ein polarer Ester, insbesondere der Methyl- oder Ethylester der Ameisensäure, Essigsäure oder Kohlensäure, Methylethylketon, tert.-Butylmethylether oder Dimethylformamid, oder ein Gemisch mehrerer dieser Lösungsmittel, ist.

5. Verfahren nach Anspruch 3 oder 4, **dadurch gekennzeichnet, dass** man als polares Lösungsmittel Methanol, Ethanol oder Isopropanol verwendet.

6. Verfahren nach Anspruch 3 oder 4, **dadurch gekennzeichnet, dass** man als polares Lösungsmittel n-Propanol verwendet.

7. Verfahren nach einem der Ansprüche 1 bis 6, **dadurch gekennzeichnet, dass** je nach Reaktionsteilnehmer bzw. Produkt (Carotinoid) sich zwischen 0,5 und 2 % seines Gewichtes in Lösung befindet.

8. Verfahren nach einem der Ansprüche 1 bis 7, **dadurch gekennzeichnet, dass** es zur Herstellung folgender Carotinoide angewandt wird:
Zeaxanthin ausgehend von [5-(4-Hydroxy-2,6,6-trimethyl-1-cyclohexenyl)-3-methyl-2,4-pentadienyl]triphenylphosphoniumchlorid und 2,7-Dimethyl-2,4,6-octatrien-1,8-dial (C₁₀-Dial);
Lycopin ausgehend von [3,7,11-Trimethyl-dodecyl-2,4,6,10-tetraenyl]triphenylphosphoniumchlorid oder -acetat, insbesondere dem (2 E/Z, 4E, 6 E,Z)-Isomeren, und C₁₀-Dial;
Carotin ausgehend von [3-Methyl-5-(2,6,6-trimethyl-1-cyclohexenyl)-2,4-pentadienyl]triphenylphosphoniumchlorid, insbesondere dem (2E, 4E)-Isomeren, und C₁₀-Dial;
Canthaxanthin ausgehend von [5-(2,6,6-Trimethyl-3-oxo-1-cyclohexenyl)-3-methyl-2,4-pentadienyl]triphenylphosphoniumchlorid und C₁₀-Dial;
Astaxanthin ausgehend von [5-(4-Hydroxy-2,6,6-trimethyl-3-oxo-1-cyclohexenyl)-3-methyl-2,4-pentadienyl] triphenylphosphoniumbromid, insbesondere dem (2E,4E)-Isomeren, und C₁₀-Dial;
Crocetindiethylester ausgehend von [3-Ethoxycarbonyl-but-2-enyl]triphenylphosphoniumchlorid und/oder -bromid, insbesondere dem (E)-Isomeren, und C₁₀-Dial;
sowie Diapo-4,4'-carotindial ausgehend vom Dimethylacetal von [7-Formyl-3,7-dimethyl-octa-2,4,6-trienyl]triphenylphosphoniumchlorid und C₁₀-Dial.

## Claims

1. A process for the manufacture of carotenoids by the Wittig reaction of a symmetrical dialdehyde with two equivalents of a phosphonium salt in one step, which process comprises carrying out the Wittig reaction in a polar reaction medium in a manner such that neither all the reactants nor the thus manufactured carotenoid are significantly dissolved in the reaction medium, whereby depending on the reactant or product (carotenoid) a maximum of 10% of its weight is present in solution.

2. A process according to claim 1, wherein a polar solvent or solvent mixture which remains monophasic on the addition of water in an amount up to about 30 vol.% and which permits the triarylphosphine oxide which is formed to dissolve well is used as the reaction medium.

3. A process according to claim 1, wherein as the polar solvents there are used alkanols having 1 to 6 carbon atoms and acetone, which are used as the sole solvents or as mixtures with one another and/or with water.

4. A process according to claim 3, wherein in each case an additional polar solvent is used, which is a polar ester, especially the methyl or ethyl ester of formic acid, acetic acid or carbonic acid, methyl ethyl ketone, tert.butyl methyl ether or dimethylformamide, or a mixture of several of these solvents.

5. A process according to claim 3 or 4, wherein methanol, ethanol or isopropanol is used as the polar solvent.

6. A process according to claim 3 or 4, wherein n-propanol is used as the polar solvent.

7. A process according to any one of claims 1 to 6, wherein depending on the reactant or product (carotenoid), between 0.5 and 2% of its weight is present in solution.

8. A process according to any one of claims 1 to 7, which is used for the manufacture of the following carotenoids:
Zeaxanthin starting from [5-(4-hydroxy-2,6,6-trimethyl-1-cyclohexenyl)-3-methyl-2,4-pentadienyl]triphenylphosphonium chloride and 2,7-dimethyl-2,4,6-octatriene-1,8-dial (C₁₀-dial);
lycopene starting from [3,7,11-trimethyl-dodecyl-2,4,6,10-tetraenyl]-triphenylphosphonium chloride or acetate, especially the (2 E/Z, 4E, 6E,Z) isomer, and C₁₀-dial;
carotene starting from [3-methyl-5-(2,6,6-trimethyl-1-cyclohexenyl)-2,4-pentadienyl]triphenylphosphonium chloride, especially the (2E,4E) isomer, and C₁₀-dial;
canthaxanthin starting from [5-(2,6,6-trimethyl-3-oxo-1-cyclohexenyl)-3-methyl-2,4-pentadienyl]triphenylphosphonium chloride and C₁₀-dial;
astaxanthin starting from [5-(4-hydroxy-2,6,6-trimethyl-3-oxo-1-cyclohexenyl)-3-methyl-2,4-pentadienyl]triphenylphosphonium bromide, especially the (2E,4E) isomer, and C₁₀-dial;
crocetin diethyl ester starting from [3-ethoxycarbonyl-but-2-enyl]triphenylphosphonium chloride and/or bromide, especially the (E) isomer, and C₁₀-dial;
and diapo-4,4'-carotenedial starting from the dimethyl acetal of [7-formyl-3,7-dimethyl-octa-2,4,6-trienyl]triphenylphosphonium bromide and C₁₀-dial.

## Revendications

1. Procédé de préparation de caroténoïdes par la réaction de Wittig d'un dialdéhyde symétrique en une étape avec deux équivalents d'un sel de phosphonium, **caractérisé en ce qu'**on met en oeuvre la réaction de Wittig dans un milieu réactionnel polaire de telle sorte que ni l'ensemble des substances participant à la réaction, ni le caroténoïde ainsi préparé, ne se dissolvent d'une manière significative dans le milieu de réaction, et où, selon les substances participant à la réaction ou le produit (caroténoïde), au plus 10 % de son poids se trouvent en solution.

2. Procédé selon la revendication 1, **caractérisé en ce qu'**on utilise en tant que milieu réactionnel un solvant ou un mélange de solvants polaires, qui reste monophasique si l'on ajoute une quantité d'eau allant jusqu'à environ 30 % en volume, et qui peut bien dissoudre l'oxyde de triarylphosphine qui se forme.

3. Procédé selon la revendication 1, **caractérisé en ce qu'**on utilise en tant que solvants polaires des alcanols ayant de 1 à 6 atomes de carbone et l'acétone, qui sont utilisés en tant que solvants uniques, ou sous forme de mélanges l'un avec l'autre et/ou avec de l'eau.

4. Procédé selon la revendication 3, **caractérisé en ce qu'**on utilise dans tous les cas un solvant polaire additionnel, qui est un ester polaire, en particulier l'ester méthylique ou éthylique de l'acide formique, de l'acide acétique ou de l'acide carbonique, la méthyléthylcétone, le tert-butylméthyléther ou le diméthylformamide, ou un mélange de plusieurs de ces solvants.

5. Procédé selon la revendication 3 ou 4, **caractérisé en ce qu'**on utilise en tant que solvant polaire le méthanol, l'éthanol ou l'isopropanol.

6. Procédé selon la revendication 3 ou 4, **caractérisé en ce qu'**on utilise en tant que solvant polaire le n-propanol.

7. Procédé selon l'une des revendications 1 à 6, **caractérisé en ce que**, selon les substances participant à la réaction ou le produit (caroténoïde), de 0,5 à 2 % de son poids se trouvent en solution.

8. Procédé selon l'une des revendications 1 à 7, **caractérisé en ce que**, pour préparer les caroténoïdes ci-après, on utilise les composés suivants :
zéaxanthine, à partir de chlorure de [5-(4-hydroxy-2,6,6-triméthyl-1-cyclohexényl)-3-méthyl-2,4-pentadiényl]triphénylphosphonium et de 2,7-diméthyl-2,4,6-octatriène-1,8-dial (C₁₀-dial) ;
lycopine, à partir de chlorure ou d'acétate de [3,7,11-triméthyl-dodécyl-2,4,6,10-tétraényl]triphénylphosphonium, en particulier de l'isomère (2 E/Z, 4E, 6 E,Z),et de C₁₀-dial ;
carotène, à partir de chlorure de [3-méthyl-5-(2,6,6-triméthyl-1-cyclohexényl)-2,4-pentadiényl]-triphénylphosphonium, en particulier de l'isomère (2E, 4E), et de C₁₀-dial ;
canthaxanthine, à partir de chlorure de [5-(2,6,6-triméthyl-3-oxo-1-cyclohexényl)-3-méthyl-2,4-pentadiényl]triphénylphosphonium et de C₁₀-dial ;
astaxanthine, à partir de bromure de [5-(4-hydroxy-2,6,6-triméthyl-3-oxo-1-cyclohexényl)-3-méthyl-2,4-pentadiényl]triphénylphosphonium, en particulier de l'isomère (2E, 4E), et de C₁₀-dial ;
ester diéthylique de la crocétine, à partir de chlorure et/ou de bromure de [3-éthoxycarbonyl-but-2-ényl]-triphénylphosphonium, en particulier de l'isomère (E), et de C₁₀-dial ;
et aussi diapo-4,4'-carotènedial, à partir de l'acétal diméthylique du chlorure de [7-formyl-3,7-diméthyl-octa-2,4,6-triényl]triphénylphosphonium et de C₁₀-dial.
